# EUROPEAN PATENT APPLICATION

(11) **EP 1 952 813 A1**
(43) Date of publication of application: **06.08.2008**
(21) Application number: 07250429.3
(22) Date of filing: 01.02.2007
(51) Int. Cl.: A61K 31/473, A61K 31/48, A61P 15/00

(54) **Medicament for the treatment of endometriosis**

(71) Applicant: Ferring International Center S.A., 1162 Saint-Prex (CH)
(72) Inventor: The designation of the inventor has not yet been filed
(74) Representative: Bates, Philip Ian

(57) **Abstract**

The use of a dopamine agonist in the manufacture of a medicament for the treatment or prevention of endometriosis.

## Description

The present invention relates to medicaments for the treatment of endometriosis.

Endometriosis may be defined as a presence of endometrial tissue, comprising both glandular epithelium and stroma, outside the uterine cavity. It is a benign gynaecological disorder, which, in a sub-population of female patients, may develop into an aggressive disease. Endometriosis is associated with various distressing symptoms including dysmenorrhoea, dyspareunia, pelvic pain and reduced fertility.

It is known that angiogenesis (the process whereby new blood vessels are formed from pre-existing vessels) may be of importance in the development of endometriosis, and that vascular permeability factor/vascular endothelial growth factor (VP/VEGF) has a role in vascular genesis, and in both physiological and pathological angiogenesis. The potential effectiveness of anti-angiogenic therapy for treating endometriosis has been assessed using a study using human endometrial tissues transplanted to immuno-compromised nude mice. Four different anti-angiogenic agents were administered three weeks after the endometrial explants had been transplanted (Nap *et al*, 2004). All four inhibitors were able to reduce the size of established explants, and new blood vessel formation was stopped. However, the known anti-angiogenic agents are highly toxic, and rather difficult to introduce in a human clinical setting.

It has now been unexpectedly found that compositions which include a dopamine agonist may be used to treat endometriosis.

The present invention therefore provides the use of a dopamine agonist in the manufacture of a medicament for the treatment and/or prevention of endometriosis. The present invention also provides the use of a dopamine agonist in the treatment and/or prevention of endometriosis. Herein, the term "treatment of endometriosis" includes treatment to reduce (or remove) the amount of endometrial tissue which is present outside the uterine cavity (e.g. reduction or removal of endometriotic lesions) and/or treatment to reduce and/or ameliorate one or more symptoms associated with endometriosis (e.g. treatment to ameliorate and/or reduce the symptoms of dysmenorrhoea). The term endometriosis includes, for example, peritoneal endometriosis, ovarian endometriosis and deep endometriosis.

Herein, the term dopamine agonist means a compound that acts like dopamine, for example, a drug which interacts with a dopamine receptor to mimic a dopamine action.

Dopamine agonists have previously been found to be useful in the treatment or prevention of ovarian hyperstimulation syndrome (OHSS) (WO 2006117608). This is a condition where the capillaries increase their vascular permeability significantly. Dopamine agonists were found to be able to reverse this effect. However, the condition of endometriosis and the potential effectiveness of anti-angiogenic therapy for treating endometriosis is not disclosed here.

Dopamine agonists within the terms of the invention include, but are not limited to, amantadine, bromocryptine, cabergoline, quinagolide, lisuride, pergolide, ropinirole and pramipexole. A preferred dopamine agonist for use in the present invention is cabergoline. A preferred dopamine agonist for use in the present invention is quinagolide. The so-called "partial dopamine agonists" (e.g. terguride) may also be used in accordance with the invention. However, the use of dopamine agonists is preferred.

Preferably, a single dopamine agonist is used.

The dopamine agonist may be administered at a dose (e.g. an oral dose to a human patient) of between 25 micrograms per day and 80 mg/day, preferably between 50 micrograms per day and 5 mg/day, more preferably between 300 micrograms per day and 1 mg/day; suitable doses within this range depend to the dopamine agonist to be used, as is readily apparent to those skilled in the art.

In a preferred embodiment, the dopamine agonist is cabergoline. Preferably the cabergoline is administered at a dose (e.g. an oral dose to a human patient) of between 0.01 and 12.5 mg/week, preferably between 0.1 and 10 mg/week, more preferably between 0.5mg and 5 mg/week, more preferably at a dose of between 3.5 mg/week and 4 mg/week. The dopamine agonist may be administered as, for example, a single daily dose (of for example, between 0.1 mg/day and 5mg/day, from 0.2 mg/day to 1mg/day, for example 0.5 mg/day); or the daily dose may be divided into two or more sub-doses to be taken at different times over a 24 hour period. The dopamine agonist (cabergoline) may be administered as a daily dose at the levels above, or as equivalent doses e.g. per week, twice a week, or every two days. In one regime, the dopamine agonist (for example, cabergoline) is administered at a total dose of between 3.5 and 12.5 mg per week (e.g. 4mg per week, 7mg per week, 10mg/week).

In another embodiment, the dopamine agonist is quinagolide. Preferably the quinagolide is administered at a dose (e.g. an oral dose to a human patient) of between 25 and 1000 micrograms/day, preferably between 25 and 500 micrograms/day, more preferably between 25 and 300 micrograms/day. The dopamine agonist may be administered as, for example, a single daily dose; or the daily dose may be divided into two or more sub-doses to be taken at different times over a 24 hour period. The dopamine agonist (quinagolide) may be administered as a daily dose at the levels above, or as equivalent doses e.g. per week, twice a week, or every two days.

In another embodiment, the dopamine agonist is bromocryptine. Preferably the bromocryptine is administered at a dose (e.g. an oral dose to a human patient) of between 10 to 80 micrograms/day, preferably 10 to 40 mg/day.

In a further embodiment, administration of a dopamine agonist may be combined with other medical or surgical treatments for endometriosis [for example, NSAIDs and/or hormonal treatments (danazol, OCs, medroxyprogesterone acetate, other progestins, GnRH agonists and antagonists, aromatase inhibitors)]. In a further embodiment, surgical treatment or medical treatment may be used prior, during or after treatment with dopamine agonist. These embodiments are discussed in a little more detail in the examples, below.

It has also been found that administration of dopamine agonist to a patient in need thereof may provide substantial clinical benefits such as, for example: significant decrease in the percentage of active endometriotic lesions; significant loss of the cellularity and organisation manifesting characteristics of atrophic or degenerative tissue in endometriotic implants; and significant decrease in the number of new blood vessels in endometriotic implants.

Medicaments based on dopamine agonists have also the advantage of high dose tolerance, with safe and well documented clinical use records.

Further, it has been found that the dopamine agonist (for example, cabergoline) may be administered for long periods of time (e.g. 1 to 3 weeks (e.g. 1 to 21 days, for example 1 to 14 days), from 1 day to 3 months, 1 day to six months, 1 day to 12 months, or 1 day to 2 years, or longer) with therapeutically beneficial effect, and low risk of side effects. The administration may be continuous at e.g. daily or weekly dose, or may be interrupted by one or more interruptions of, for example, a number (1 to 3) of weeks or a number (1 to 3) of months. The dopamine agonist may be administered for as long as pain (or other symptom) continues.

The dopamine agonist (for example, cabergoline, quinagolide) may be administered to a pregnant subject.

In an example of the invention, vascular endothelial growth factor (VEGF) may be targeted by the dopamine agonist, as a factor in the development of endometriosis. Other mechanisms of action of the dopamine agonist are within the scope of the invention.

The dopamine agonist is administered as a pharmaceutically acceptable preparation. Preparations may be administered in accordance with the invention in pharmaceutically acceptable compositions that may optionally comprise pharmaceutically acceptable salts, buffering agents, preservatives and excipients. Pharmaceutical preparations which include dopamine agonist(s) as active agents are well known in the art and are commercially available. For example, cabergoline is available under the registered trade marks Cabaser and Sogilen/Dostinex. The use of such commercially available dopamine agonist preparations in the treatment of endometriosis is according to the invention.

The mode of administration selected will depend on the acuteness and severity of the condition being treated, and the dosage required. Any mode of administration that produces desired therapeutic effect without unacceptable adverse effects is relevant in practicing the invention. Such modes of administration may include oral, rectal, topical, transdermal, sublingual, intramuscular, parenteral, intravenous, intracavity, vaginal, and adhesive matrix to be used during surgery. Various approaches for formulating compositions for use in accordance with the invention are described in the Handbook of Pharmaceutical Excipients, Third Edition, American Pharmaceutical Association, USA and Pharmaceutical Press UK (2000), and Pharmaceutics - The Science of Dosage Form Design, Churchill Livingston (1988).

In a preferred embodiment, the administration is oral. Compositions suitable for oral administration include capsules, cachets, tablets, syrups, elixirs or lozenges.

According to the present invention in a further aspect, there is provided a method of treatment or prevention of endometriosis comprising a step of administration to a patient in need thereof of a dopamine agonist.

Preferably the dopamine agonist is administered in the form of a pharmaceutical preparation which includes one or more dopamine agonists as the active ingredient.

The dopamine agonist may be administered at a dose (e.g. an oral dose to a human patient) of between 25 micrograms per day and 80 mg/day, preferably between 50 micrograms per day and 5 mg/day, more preferably between 300 micrograms per day and 1 mg/day; suitable doses within this range depend to the dopamine agonist to be used, as is readily apparent to those skilled in the art.

In a preferred embodiment, the dopamine agonist is cabergoline. Preferably the cabergoline is administered at a dose (e.g. an oral dose to a human patient) of between 0.01 and 12.5 mg/week, preferably between 0.1 and 10 mg/week, more preferably between 0.5mg and 5 mg/week, more preferably at a dose of between 3.5 mg/week and 4 mg/week. The dopamine agonist may be administered as, for example, a single daily dose (of for example, between 0.1 mg/day and 5mg/day, from 0.2 mg/day to 1 mg/day, for example 0.5 mg/day); or the daily dose may be divided into two or more sub-doses to be taken at different times over a 24 hour period. The dopamine agonist (cabergoline) may be administered as a daily dose at the levels above, or as equivalent doses e.g. per week, twice a week, or every two days. In one regime, the dopamine agonist (for example, cabergoline) is administered at a total dose of between 3.5 and 12.5 mg per week (e.g. 4mg per week, 7mg per week, 10mg/week).

In another embodiment, the dopamine agonist is quinagolide. Preferably the quinagolide is administered at a dose (e.g. an oral dose to a human patient) of between 25 and 1000 micrograms/day, preferably between 25 and 500 micrograms/day, more preferably between 25 and 300 micrograms/day. The dopamine agonist may be administered as, for example, a single daily dose; or the daily dose may be divided into two or more sub-doses to be taken at different times over a 24 hour period. The dopamine agonist (quinagolide) may be administered as a daily dose at the levels above, or as equivalent doses e.g. per week, twice a week, or every two days.

In another embodiment, the dopamine agonist is bromocryptine. Preferably the bromocryptine is administered at a dose (e.g. an oral dose to a human patient) of between 10 to 80 mg/day, preferably 10 to 40 mg/day.

The administration of a dopamine agonist may be combined with other medical or surgical treatments for endometriosis [for example, NSAIDs and/or hormonal treatments (danazol, OCs, medroxyprogesterone acetate, other progestins, GnRH agonists and antagonists, aromatase inhibitors)]. In a further embodiment, surgical treatment or medical treatment may be used prior, during or after treatment with dopamine agonist.
The applicants have found that the dopamine agonist (for example, cabergoline) may be administered for long periods of time (e.g. 1 to 3 weeks (e.g. 1 to 21 days, for example 1 to 14 days), from 1 day to 3 months, 1 day to six months, 1 day to 12 months, or 1 day to 2 years, or longer) with therapeutically beneficial effect, and low risk of side effects. The administration may be continuous at e.g. daily or weekly dose, or may be interrupted by one or more interruptions of, for example, a number (1 to 3) of weeks or a number (1 to 3) of months. The dopamine agonist may be administered for as long as pain (or other symptom) continues.

The patient may be pregnant.

The present invention will now be illustrated with reference to the Examples and attached drawings, in which:
FIGURE 1 shows the percentage of active lesions following the animal study discussed below, for the control group, and the groups treated with low dose (0.05 mg/kg/day) and high dose (0.1 mg/kg/day) of cabergoline;
FIGURE 2 shows the blood vessels (mm³) for the control and low and high dose groups; and
FIGURE 3 shows the percentage of "mature" and "newly formed" blood vessels in the animals of the control, low and high dose groups.

### Example 1

An experimental animal endometriosis model was developed in nude mice by inserting human endometrium fragments. Female mice (Hsd: Athimic Nude-*nu,* Harlan Ibérica S.L, Barcelona, Spain) were individually housed in autoclaved cages and bedding, in laminar flow filtered hoods. The animal room was maintained at 26 C with a 12-h light, 12-h dark cycle, and mice were fed *ad libitum* with autoclaved laboratory rodent chow and acidified water. All handling was performed in laminar flow filtered hoods. A mixture of ketamine/medetomidine (75 µg/g ketamine and 1 µg/g medetomidine) (Ketolar^{®}, Parke-Davis, España; Domtor^{®}, Pfizer, Spain) i.p injected, was used to anesthetize mice before invasive procedures and atipamezole (Antisedan^{®}, SmithKline Beecham, Spain) 1 µg/g i.p injected, to reverse the anesthesia effects, was used after invasive procedures, using sterile instruments.

At the age of 5 weeks, sterile 60-d release capsules containing 18 mg 17β-estradiol (Innovative Research of America, Sarasota, FL) were placed sc in the neck of each animal. According to the manufacturer's information, capsules provide continuous release of hormone at serum concentrations of 150-250 pmol/liter, in the range of physiological levels in mice during the estrous cycle. This stable physiological level of estrogen promotes the growth of transplanted human endometrium and avoids intermouse differences related to various stages of the estrous cycle.

Four days after insertion of the estrogen pellet, an entrance was made to the peritoneal cavity in the midline in the lower abdomen to insert fresh human endometrium from ovum pick-up donors. These fragments were stuck by glue (Vetabond®, 3M Animal Care products, USA) to the peritoneum. This protocol allowed to mimic the pathologic situation of retrograde menstruation that occur in women with endometriosis.

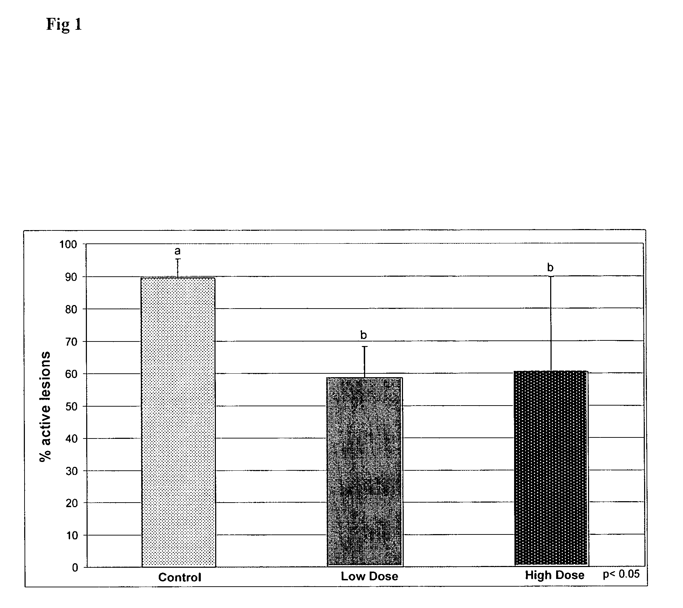

Three weeks after implantation the animals were split into three groups. The first was a control group; the second was the low dose group, treated with 0.05 mg/kg/day oral cabergoline; and a high dose group, treated with an oral dose of 0.1 mg/kg/day cabergoline. Two weeks after treatment the animals were sacrificed and the endometriotic lesions sampled and analysed. The results are shown in Figs. 1, 2 and 3.

The anti-angiogenic effects of the dopamine agonists were assessed by by immunofluorescence, the antibodies used in confocal microscopy raised against Von Willebrand factor (vWF monoclonal IgG1, DAKO Corp., Denmark) present in endothelial cells and vascular smooth muscle cells (monoclonal IgG2 α-SMA-FITC conjugated de Sigma, St Louis, USA). Morphometric study was performed to measure the implants area and cellular density. Immunocytochemistry with Ki-67 (monoclonal IgG1 DAKO Corp., Denmark) antibody was performed in order to evaluate the proliferative activity of the implants. The histopathological and subcellular ultrastructural changes were detected using an optical microscope, transmission electronic microscope and histochemical staining.

### Toxicity

No mice in the study died following the administration of cabergoline. Cabergoline did not appear to change the overall health of the mice, because the body of weight of the mice in the different treatment groups was not significantly different (results not shown).

### Results

Figure 1 shows the percentage of active lesions following the animal study discussed below, for the control group, and the groups treated with low dose (0.05 mg/kg/day) and high dose (0.1 mg/kg/day) of cabergoline. The animals treated with cabergoline (both low and high dose groups) have significantly fewer active lesions when compared to the control group. In other words, treatment with cabergoline appears to reduce the number of active endometriotic lesions in this model.

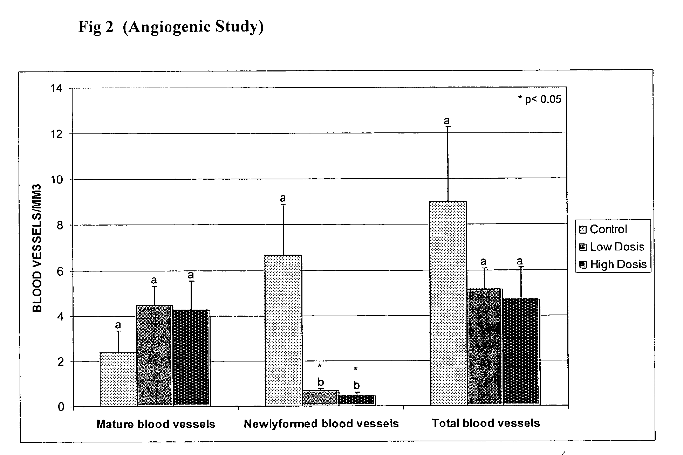

Figure 2 shows the blood vessels (mm³) for the control, low and high dosage groups, divided between "mature" blood vessels and "newly formed" blood vessels. The control group has a greater proportion of newly formed blood vessels (indicative of significant angiogenesis), while the low and high dose groups have a significantly greater proportion of mature blood vessels, suggestive of significantly reduced angiogenesis. This was also demonstrated by histology (results not shown). These results indicate that the cabergoline significantly reduced new blood vessel formation (angiogenisis), in this model.

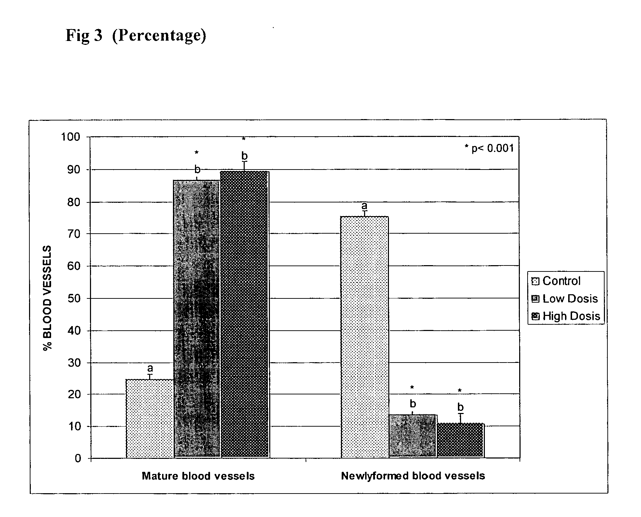

Figure 3 shows the percentage of blood vessels in the control and low and high dosage groups. The control group has approximately 74% of the total blood vessels as newly formed blood vessels, indicating significant angiogenisis. The low and high dosage groups, on the other hand, have approximately 85 to 89% of the total as mature blood vessels, indicating that angiogenisis is not significant. These results indicate that the cabergoline significantly reduced new blood vessel formation (angiogenisis), in this model.

The implants presented a higher cellular stroma and the histological aspect of complete reorganization and structure typically seen in endometriosis lesion in the control group, whereas the implants in mice included in the low and high dosage groups showed a lax stroma with loose cellularity and organization, which is characteristic of atrophic/degenerative tissue. Morphometry showed no difference in cellular density and stroma/glands glands area among groups.

The results indicate that the dopamine agonist cabergoline, administered at a dose of 0.05 and 0.1 mg/kg/day, was able to:
(a) significantly decrease the number of active endometriotic lesions;
(b) cause loss of cellularity and organisation presenting characteristics in atrophic or degenerative tissue in the endometriotic implants; and
(c) significantly decrease the number of new blood vessels in the endometrioic implants.
There is a high homology between the human and rodent VEGF systems and it is therefore indicated that the activity shown in the above rodent model is applicable to human models. The results indicate that administration of dopamine agonists has a significant effect on endometriosis, possibly linked to action on angiogenesis.

### Example 2

A formulation as a tablet for oral use is 0.5 mg of cabergoline (commercially available as Dostinex®, Pfizer, Spain).

### Further Examples

Example A: A patient undergoes diagnostic laparoscopy after suffering chronic pelvic pain and is diagnosed with endometriosis type III. At the same laparoscopy, the patient undergoes surgery such as resection of available lesions and administration of cabergoline is initiated.
Example B: Patient diagnosed previously of endometriosis, presenting with symptoms of pelvic pain and dysmenorrhea. Administration of cabergoline is initiated without surgery.
Example C: Patient diagnosed with endometriosis undergoing treatment with GnRH agonists (or danazol or aromatase inhibitors) and administration of cabergoline is initiated (with continued use of GnRH agonist) for a period. After a further 3 or 6 months of no therapy, patient restarts cabergoline for a further period.

## Claims

1. Use of a dopamine agonist in the manufacture of a medicament for the treatment or prevention of endometriosis.

2. A dopamine agonist for use in the treatment or prevention of endometriosis.

3. The use according to claim 1 or claim 2, wherein said dopamine agonist is one of amantadine, bromocryptine, cabergoline, quinagolide, lisuride, pergolide, ropinirole and pramipexole.

4. The use according to any preceding claim wherein the dopamine agonist is cabergoline administered at a dose of between 0.01 and 12.5mg/week.

5. The use according to any preceding claim wherein the dopamine agonist is quinagolide administered at a dose of between 25 and 1000 micrograms/day.

6. The use according to any preceding claim wherein the dopamine agonist is bromocryptine administered at a dose of between 10 and 80mg/day.

7. The use according to any preceding claim wherein the dopamine agonist is administered for a period of between 1 day and 2 years.

8. The use according to any preceding claim, wherein the dopamine agonist is administered in combination with other surgical or medicinal treatment for endometriosis.

9. The use according to any preceding claim wherein the dopamine agonist (s) is used for the treatment or prevention of endometriosis in a pregnant subject.

10. The use according to any preceding claim, in which vascular endothelial growth factor (VEGF) is targeted.

11. The use according to any preceding claim wherein the administration is oral, and in the form of compositions as capsules, cachets, tablets, syrups, elixirs or lozenges.

12. A method of treatment or prevention of endometriosis comprising a step of administration to a patient in need thereof of a dopamine agonist.
